# FASCICULE DE BREVET EUROPEEN

(11) **EP 1 042 663 B1**
(45) Date de publication et mention de la délivrance du brevet: **20.08.2003**
(21) Numéro de dépôt: 98958962.7
(22) Date de dépôt: 07.12.1998
(51) Int. Cl.: G01N 21/07

(54) **DISPOSITIF, PROCEDE ET APPAREIL DE MISE EN OEUVRE DU PROCEDE, POUR EFFECTUER UN DOSAGE D'AU MOINS UN COMPOSANT PARTICULIER DANS UN ECHANTILLON DE PRODUIT**
VORRICHTUNG, VERFAHREN UND GERÄT ZUR DURCHFÜHRUNG DES VERFAHRENS, FÜR DIE DOSIERUNG VON MINDESTENS EINER BESONDEREN KOMPONENTE EINER PRODUKTPROBE
DEVICE, METHOD AND APPARATUS FOR IMPLEMENTING THE METHOD, FOR DOSING AT LEAST A PARTICULAR CONSTITUENT IN A PRODUCT SAMPLE

(30) Priorité: 31.12.1997 FR 9716787
(43) Date de publication de la demande: 11.10.2000
(73) Titulaire: Stago International, 92600 Asnières (FR)
(72) Inventeur: ROUSSEAU, Alain, F-75004 Paris (FR); CANTON, Michel, F-13260 Cassis (FR)
(74) Mandataire: Schrimpf, Robert
(86) Numéro de dépôt international: FR9802639
(87) Numéro de publication internationale: WO99035483

(56) Documents cités:
- EP-A- 0 262 497
- EP-A- 0 339 277
- WO-A-94/25159
- US-A- 4 652 137
- US-A- 5 599 668

## Description

La présente invention concerne un dispositif destiné à effectuer un dosage qualitatif ou quantitatif d'au moins un composant particulier dans un échantillon de produit, de préférence permettant de mettre en oeuvre un dosage immunologique, ainsi qu'un procédé et un appareillage destinés à mettre en oeuvre le dispositif.

De nombreuses méthodes ont été développées pour l'identification, la détection ou la quantification d'analytes dans des échantillons chimiques ou biologiques.

Ces méthodes sont pour la plupart basées sur la formation de complexes par réaction d'affinité entre membres d'une paire de liaison spécifique.

Ces réactions, de type ligand/récepteur, résultent par exemple d'interactions entre un antigène et un anticorps spécifique, d'une hybridation entre deux séquences d'acides nucléiques complémentaires ou d'un phénomène de reconnaissance entre le site de liaison d'une protéine, par exemple une enzyme, hormone, ou autre entité biologique, et son ligand, substrat ou récepteur.

La formation d'un complexe d'affinité permet de mettre en évidence la présence de l'analyte recherché dans l'échantillon. Cet analyte peut éventuellement être quantifié, s'il est possible de séparer les formes complexées de celles restées à l'état libre, ou de mesurer le taux d'occupation des ligands spécifiques de l'analyte.

Ce type de méthode de détection et quantification d'un analyte présent dans un échantillon, parfois à l'état de trace, offre un grand intérêt pour les laboratoires de recherche ou d'analyse, notamment les laboratoires d'analyse clinique ou biologique.

Toutefois, pour une utilisation en routine, les méthodes doivent pouvoir être appliquées simultanément sur un grand nombre d'échantillons. En outre, pour un même échantillon, il est souvent nécessaire de réaliser plusieurs tests.

De ce fait, dans la plupart des cas, les protocoles manuels d'analyse de routine font intervenir plusieurs réactions et étapes de manipulation successives. Ces tests multiples sont réalisés sur des échantillons en série, dans des très grands centres dans lesquels il arrive que plusieurs dizaines de milliers d'échantillons soient testés par jour. De tels tests multiples peuvent de ce fait imposer des contraintes et nécessiter des temps de réalisation relativement longs. De plus, les manipulations successives qu'ils nécessitent, peuvent donner lieu à des erreurs dans les résultats.

Le problème de l'automatisation de ce type de test s'est donc rapidement posé, et différents dispositifs ont ainsi été élaborés pour parvenir à une automatisation, ou, tout au moins, une simplification des étapes successives indiquées ci-dessus.

Ces dispositifs restent toutefois pour la plupart relativement complexes ou adaptés à la détection d'un type d'analyte particulier (cellule ou molécule) ou bien encore ne permettent de réaliser que des analyses qualitatives. De tels dispositifs sont notamment décrits dans les documents EP 0339277 et EP 0426729.

En particulier, le document EP 339 277 décrit un dispositif pour effectuer des réactions analytiques successives pour le dosage d'un analyte dans un échantillon liquide d'essai faisant intervenir des réactions analytiques entre l'analyte et des réactifs analytiques qui interagissent avec l'analyte pour la production d'une réponse détectable en fonction de l'analyte.

Ce dispositif comporte un récipient fermé ayant un axe de rotation horizontal. Ce récipient fermé est délimité extérieurement par une paroi cylindrique et comporte intérieurement deux parois concentriques en forme de cuillère qui définissent entre elles une zone d'admission de l'échantillon. Entre lesdites parois en forme de cuillère et la paroi périphérique cylindrique, il est défini plusieurs zones réactionnelles auxquelles sont incorporés les réactifs analytiques spécifiques.

Selon ce document, l'échantillon est introduit par une voie d'entrée dans la chambre d'admission définie entre les parois en forme de cuillère et s'ouvrant vers les zones réactionnelles. En faisant pivoter ledit récipient suivant un mouvement pendulaire autour de son axe horizontal, l'échantillon liquide est transporté par gravité dans les zones réactionnelles où il interagit avec les réactifs puis transporté jusqu'à une zone d'examen située au centre du récipient.

Un tel dispositif est principalement conçu pour éviter toute centrifugation du produit lors de la réalisation du dosage.

On connaît également du document JP HEI - 5 215 750 un dispositif pour la détection et l'analyse de populations cellulaires, qui comporte une platine circulaire horizontale montée à rotation autour d'un axe vertical. Cette platine ouverte est recouverte d'anticorps.

Elle est entrainée en rotation autour de son axe vertical de sorte que l'échantillon introduit en son centre est reparti sous l'action d'une force centrifuge sur ladite platine.

Les étapes de lavage ultérieures sont réalisées de la même manière en introduisant au centre de la platine le liquide de rinçage qui, lors de la rotation de cette dernière s'évacue vers sa périphérique en rinçant la surface sur laquelle le composant à doser est fixé. Afin de récupérer le liquide de rinçage, il est prévu un récipient placé en dessous de la platine.

Enfin, on connaît du document WO 94 25 159 un dispositif pour effectuer un dosage qualitatif et/ou quantitatif d'un composant particulier dans un échantillon de produits, qui comporte un conteneur sensiblement circulaire monté à rotation sur un arbre d'entraînement par un logement central, et dans lequel sont ménagées des chambres tests, qui s'étendent selon des rayons du conteneur et qui comportent un gradient de densité. Dans la partie centrale du conteneur, il est prévu une chambre annulaire de centrifugation qui est en communication avec chaque chambre test.

Cette chambre annulaire de centrifugation peut être divisée en deux parties qui communiquent entre-elles par une ouverture supérieure.

La paroi de délimitation de la première partie de la chambre de centrifugation, est inclinée de sorte que le transfert de produits de la première partie vers la deuxième partie s'effectue par débordement au-delà de la paroi de délimitation, via l'orifice de communication. De même, la paroi de délimitation de la deuxième partie de la chambre de centrifugation présente une pente inclinée de sorte que le tranfert du mélange dans chaque chambre test s'effectue par débordement via l'orifice de communication entre la deuxième partie de la chambre de centrifugation et lesdites chambres tests.

La pente de la paroi de délimitation la deuxième partie de la chambre de centrifugation est supérieure à la pente de la paroi de délimitation de la première partie de sorte que lors de la centrifugation, le produit passe tout d'abord de la première partie de la chambre de centrifugation située près de l'axe de rotation du conteneur, vers la deuxième partie de la chambre de centifugation avant d'être transféré vers les chambres tests.

L'invention propose un nouvel agencement d'un dispositif de dosage de conception simple et d'utilisation facile qui peut être manipulé individuellement avec un nombre minimum de manipulations pour réaliser un dosage, permettant la réalisation de dosages à proximité du lieu de prélèvement de l'échantillon de produit contenant le composant particulier à doser, un tel dispositif présentant un agencement optimisé et permettant en outre de réaliser des essais en série à partir de faibles quantités d'échantillons.

Plus particulièrement, selon l'invention, il est proposé un dispositif pour effectuer un dosage qualitatif et/ou quantitatif d'au moins un composant particulier dans un échantillon de produit par marquage et fixation, ledit dispositif comprenant un conteneur et un couvercle assemblés pour former un récipient fermé.

Ce dispositif est caractérisé en ce que ledit récipient fermé présente un axe vertical, en ce que le conteneur et le couvercle portent des parois cylindriques coaxiales qui lors de l'assemblage de ces derniers se positionnent par paire l'une contre l'autre en délimitant au moins trois chambres annulaires concentriques à l'intérieur du récipient, à savoir depuis l'axe une chambre d'admission destinée à recevoir l'échantillon et le cas échéant permettant le marquage du composant, une chambre de fixation et de lecture dudit composant marqué et une chambre d'évacuation, en ce que les parois cylindriques coaxiales formant des séparations entre les chambres annulaires successives comprennent chacune au moins une ouverture, et en ce que le couvercle et le conteneur assemblés sont aptes à tourner l'un par rapport à l'autre autour de l'axe vertical et les ouvertures des parois cylindriques coaxiales du conteneur et du couvercle sont placées à des positions angulaires déterminées, de manière que par déplacement l'une par rapport à l'autre des parois cylindriques de chaque paire, les ouvertures de chaque paire de parois sont aptes à être positionnées en regard l'une de l'autre ou de manière décalée angulairement, pour mettre en communication ou isoler l'une de l'autre les chambres annulaires successives.

Selon un agencement préférentiel du dispositif conforme à l'invention, lesdites ouvertures prévues dans les parois cylindriques coaxiales du conteneur et du couvercle, sont positionnées de telle manière que les ouvertures d'une paire de parois cylindriques sont en regard l'une de l'autre pour mettre en communication deux chambres annulaires successives, les ouvertures des autres paires de parois cylindriques sont positionnées de manière décalée angulairement de sorte que les autres chambres annulaires sont isolées.

Suivant d'autres caractéristiques avantageuses du dispositif conforme à l'invention, il comporte sur le couvercle et sur le conteneur des moyens de positionnement indexé de ces derniers. Le couvercle comporte un téton sur la face externe d'une de ces parois cylindriques coaxiales situées à l'extérieur des autres parois cylindriques de façon à former le bord périphérique externe du récipient, ledit téton formant une prise ou un appui pour faire tourner le couvercle autour de l'axe vertical par rapport au conteneur. Le dispositif selon l'invention comporte un orifice central traversant isolé de la chambre annulaire immédiatement adjacente et destiné à s'enfiler sur un arbre vertical d'entraînement en rotation pour la mise en rotation dudit récipient.

Dans le fond du couvercle ou dans le fond du conteneur, il peut être prévu un orifice d'entrée dans la chambre d'admission.

Selon une variante du dispositif conforme à l'invention, il peut être prévu que le récipient comporte entre la chambre d'admission et la chambre d'évacuation plusieurs chambres de fixation et de lecture concentriques.

Le dispositif selon l'invention présente une forme ergonomique optimisée. Plus particulièrement, son récipient présente la forme d'un disque.

Avantageusement, le conteneur du dispositif selon l'invention est réalisé en un matériau transparent de manière à permettre une lecture des composants marqués fixés dans la chambre de fixation et de lecture au travers des parois dudit récipient. Le couvercle peut être opacifié ou être traité de façon à éviter des rayonnements parasites, la lecture pouvant être effectuée à l'aide d'une caméra CCD.

Le dispositif selon la présente invention comporte de préférence dans la chambre de fixation au moins un récepteur du composant à doser, ledit récepteur étant fixé dans la chambre. Il faut comprendre selon la présente invention que l'on désignera par récepteur et ligand de façon générique deux éléments qui sont liés par des interactions fortes, il peut donc s'agir aussi bien du couple antigènes/anticorps que du couple acide nucléique/acide nucléique complémentaire ou bien ligand vrai et récepteur ou autres interactions fortes.

Les techniques permettant de fixer des protéines (antigènes, anticorps par exemple) ou des acides nucléiques sur des surfaces en matière plastique ou même en verre sont bien connues de l'homme du métier, il s'agit de technologies actuellement utilisées notamment pour fixer ce même type de composants dans les plaques standards à cupules qui sont utilisées par exemple dans l'ELISA ou bien qui pourront être adaptées en fonction du type de matière polymère mis en oeuvre.

De préférence le récepteur sera fixé sur le fond du conteneur, si possible sous forme d'une monocouche de façon à permettre une lecture plus aisé. En effet, lorsque le moyen de lecture est une caméra CCD, le rayonnement traversera le fond du conteneur et sera ou non modifié par la présence d'un composant marqué puis sera récupéré après un deuxième passage au travers du fond du conteneur.

La technique mise en oeuvre s'apparente de façon générale à des procédés de dosage immunologique dit sandwich, c'est-à-dire que l'élément à détecter réagit avec le récepteur par exemple l'anticorps et est lui-même marqué par un autre élément le reconnaissant, qui porte un marquage aussi bien physique, c'est-à-dire des particules, ou bien chimique par exemple à l'aide d'éléments fluorescents ou qui peuvent être rendus fluorescents.

A ce sujet, selon une caractéristique avantageuse de l'invention, les particules de marquage présentent un diamètre suffisant, de préférence supérieur ou égal à environ 100 fois le diamètre du ou des composants à doser et possèdent des propriétés optiques permettant leur détection par comptage.

On entend par « possèdent des propriétés optiques » le fait que lesdites particules sont aptes à réfléchir tout ou partie d'un rayonnement lumineux émis par un système de détection, en direction desdites particules.

Bien qu'il soit possible de marquer le composant avant son introduction dans le dispositif, on préféra que l'élément de marquage susceptible de marquer le composant à doser soit placé dans la chambre d'admission, par exemple sous une forme sèche non fixée, il pourra s'agir par exemple d'anticorps marqués reconnaissant l'un des épitopes de l'antigène à doser, un autre anticorps étant fixé dans la chambre de fixation et de marquage.

Dans le dispositif selon la présente invention, le récepteur fixé est choisi comme cela a été indiqué précédemment parmi :
- les anticorps, les antigènes, les séquences d'acides nucléiques complémentaires,
- les récepteurs vrais, pour doser spécifiquement des composants qui sont :
- les antigènes, les anticorps, les séquences d'acides nucléiques, les ligands desdits récepteurs.

On utilisera de préférence pour les dosages immunologiques, un antigène ou un anticorps marqué pour doser respectivement l'élément complémentaire et on fixera dans la chambre de fixation et de lecture un autre élément complémentaire.

Il est également possible de prévoir un dosage multiple permettant de doser éventuellement plusieurs antigènes ou plusieurs anticorps. Il suffit pour ce faire que la chambre de fixation et de lecture soit divisée en pluralité de secteurs angulaires sur lesquels sont fixés des récepteurs différents les uns des autres, destinés chacun à la fixation et à la lecture d'un composant marqué différent. A cet effet, il peut être particulièrement avantageux selon l'invention, qu'un secteur angulaire de la chambre de fixation soit laissé exempt de récepteurs, de manière à constituer un secteur blanc destiné à la réalisation d'une lecture d'initialisation dudit dispositif pour faire le zéro.

La lecture pourra être réalisée selon l'invention grâce à une caméra CCD laquelle pourra être asservie bien entendu à un dispositif informatique qui permettra de reconstituer le dosage de chacun des éléments en fonction des lectures qui seront faites sur les différents secteurs.

Selon une caractéristique particulièrement avantageuse du dispositif selon l'invention, la caméra CCD est apte à compter de manière discrétionnaire par émission/réception d'un signal lumineux, le nombre des composants marqués fixés dans chaque chambre de fixation et de lecture, de façon à obtenir un signal de détection numérique.

Cela passe par l'utilisation de particules ou microsphères pour marquer le ou les composants à doser dans l'échantillon, ces particules présentant préférentiellement une taille supérieure à environ 100 fois la taille des molécules recherchées, et étant capables de renvoyer tout ou partie d'un rayonnement lumineux émis dans leur direction, rayonnement réfléchi constituant autant d'événements qui sont captés par la caméra CCD utilisée et transmis à un outil informatique adapté pour exprimer en nombre absolu et en temps réel les événements détectés.

L'invention propose également un procédé de dosage qualitatif et/ou quantitatif d'au moins un composant particulier dans un échantillon de produit par marquage et fixation.

Ce procédé est caractérisé en ce qu'on utilise au moins un dispositif selon l'invention qui contient des récepteurs spécifiques du composant à doser fixés dans chaque chambre de fixation et de lecture, et dans lequel
a) on place dans la chambre d'admission isolée des autres chambres annulaires, l'échantillon de produit contenant le composant marqué
b) on fait tourner le couvercle par rapport au conteneur de manière à mettre en communication la chambre d'admission avec chaque chambre de fixation et de lecture, la chambre d'évacuation étant isolée des autres chambres annulaires,
c) on entraîne en rotation le dispositif autour de son axe vertical de manière à disperser par centrifugation dans chaque chambre de fixation et de lecture l'échantillon de produit contenant le composant marqué, ce dernier venant alors se lier par interaction forte aux récepteurs spécifiques fixés dans chaque chambre de fixation et de lecture,
d) on fait tourner le couvercle par rapport au conteneur de manière à mettre en communication la chambre de fixation et de lecture avec la chambre d'évacuation,
e) on entraîne en rotation le dispositif autour de son axe de manière à disperser par centrifugation le surplus d'échantillon dans la chambre d'évacuation,
f) on rince l'intérieur du dispositif à l'aide d'un liquide de rinçage que l'on fait circuler par centrifugation dans les différentes chambres annulaires dudit dispositif en reproduisant les étapes b), c), d) et e) précédentes de manière à garder dans chaque chambre de fixation et de lecture seulement le composant marqué lié par interaction forte aux récepteurs fixés,
g) on détecte et on dose au travers de la ou les paroi(s) dudit dispositif ledit composant marqué.

Le procédé selon l'invention est automatisable et « générique », il peut être appliqué à la détection et au comptage de substances cibles, aussi bien de nature moléculaire que particulaire, vésiculaire ou cellulaire.

L'invention propose également un appareil pour le mise en oeuvre du procédé précité, caractérisé en ce qu'il comprend un arbre vertical d'entraînement en rotation sur lequel sont enfilés des dispositifs selon l'invention, des moyens pour maintenir à distance les uns des autres lesdits dispositifs, des moyens pour entraîner en rotation dans les deux sens ledit arbre vertical d'entraînement, des moyens d'injection d'échantillons de produit et du liquide de rinçage dans les chambres d'admission desdits dispositifs enfilés sur l'arbre d'entraînement, et des moyens pour faire tourner les couvercles des dispositifs relativement aux conteneurs de manière mettre en communication ou à isoler les différentes chambres annulaires successives de ces derniers, et un moyen de lecture des agents marqués fixés.

Ainsi, grâce à l'appareil selon l'invention, on peut avantageusement réaliser automatiquement, selon le procédé conforme à l'invention, en utilisant le dispositif selon l'invention, des dosages de différents composants d'un même échantillon, ou un même composant en particulier dans plusieurs échantillons de produits différents.

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue de dessus du couvercle d'un mode de réalisation du dispositif selon l'invention,
- la figure 2 est une vue en coupe selon le plan A-A de la figure 1,
- la figure 3 est une vue de dessus du conteneur du mode de réalisation de la figure 1 du dispositif selon l'invention,
- la figure 4 est une vue en coupe selon le plan A-A de la figure 3.
- la figure 5 présente deux courbes de détection d'un composant donné dans un sérum, par fluorescence et par comptage de microsphères de marquage selon l'invention, et
- la figure 6 présente deux courbes de détection d'un composant donné dans un sérum, par la méthode ELISA et par comptage de microsphères selon l'invention.

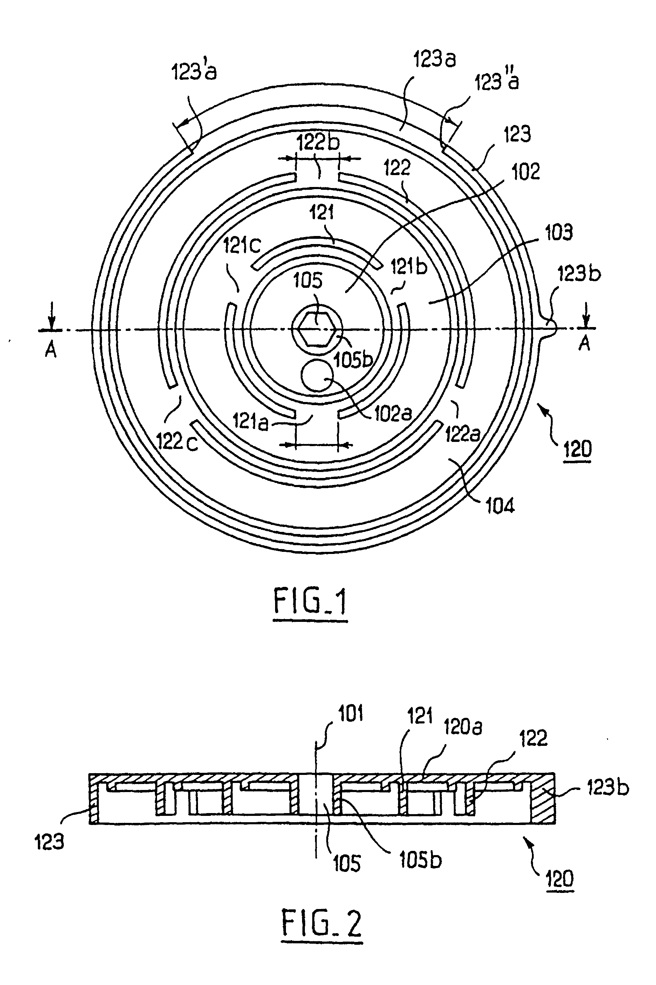

Sur les figures 1 à 4, on a représenté en pièces détachées, un dispositif pour effectuer un dosage qualitatif et/ou quantitatif d'au moins un composant particulier dans un échantillon de produit par marquage et fixation.

Un tel dispositif est utilisé avantageusement pour réaliser un dosage immunologique, une détection de micro-organismes, un dosage de polluants ou encore pour mettre en évidence une séquence d'acide nucléique particulière.

Un tel dispositif comprend un conteneur 110 et un couvercle 120 destinés à être assemblés pour former un récipient fermé.

Comme cela est représenté sur les figures, le conteneur 110 et le couvercle 120 présentent une forme générale circulaire, avec un axe central de symétrie 101, de sorte que lorsqu'ils sont assemblés, ledit récipient fermé ainsi formé présente la forme d'un disque avec un axe vertical 101.

Le conteneur 110 et le couvercle 120 présentent chacun un fond 110a, 120a qui porte des parois cylindriques coaxiales 111, 112, 113 ; 121, 122, 123 (ici au nombre de trois). Lors de l'assemblage dudit conteneur 110 et dudit couvercle 120 ces parois cylindriques coaxiales se positionnent par paire l'une contre l'autre en délimitant ici trois chambres annulaires concentriques 102, 103, 104 à l'intérieur du récipient fermé.

En partant de l'axe vers l'extérieur, lesdites parois cylindriques délimitent tout d'abord une chambre d'admission 102 destinée à recevoir l'échantillon de produit et le cas échéant permettant le marquage du composant à doser, par exemple un anticorps marqué par une particule sous forme sèche, une chambre de fixation et de lecture dudit composant marqué 103, comportant par exemple un anticorps fixé au fond du conteneur, et une chambre d'évacuation 104.

Selon le mode de réalisation représenté, le couvercle présente un diamètre légèrement supérieur au diamètre du conteneur, de sorte que le couvercle se positionne sur le conteneur. La paroi cylindrique 123 du couvercle 120 située à l'extérieur des autres parois cylindriques coaxiales 121, 122 forme alors selon le mode de réalisation représenté, le bord périphérique extérieur dudit récipient. Les parois cylindriques coaxiales 111, 112, 113 du conteneur 110 sont destinées à venir se positionner contre les faces intérieures des parois cylindriques coaxiales 121, 122, 123 du couvercle 120.

Les parois intérieures cylindriques coaxiales 111, 112 et 121, 122 du conteneur 110 et du couvercle 120 qui forment des séparations entre les chambres annulaires successives 102, 103, 104 comprennent chacune au moins une ouverture 111a, 112a et 121a, 122a.

Ici chacune de ces parois cylindriques coaxiales 111, 112 ; 121, 122 comprend trois ouvertures 111a, 111b, 111c, 112a, 112b, 112c ; 121a, 121b, 121c ; 122a, 122b, 122c réparties régulièrement sur le pourtour de chaque paroi en étant décalées deux à deux d'environ 120 degrés.

Dans l'exemple de réalisation représenté, les ouvertures des parois cylindriques coaxiales du conteneur 110 et du couvercle 120 sont formées par des encoches.

Lesdites ouvertures des parois cylindriques coaxiales du conteneur 110 et du couvercle 120 sont placées à des positions angulaires déterminées, et le couvercle 120 et le conteneur 110 assemblés sont aptes à tourner l'un par rapport à l'autre autour de l'axe vertical 101 de manière que par déplacement l'une par rapport à l'autre des parois cylindriques de chaque paire formant une séparation de chambres annulaires successives, lesdites ouvertures de chaque paire des parois sont aptes à être positionnées en regard l'une de l'autre ou de manière décalée angulairement, pour mettre en communication ou isoler l'une de l'autre lesdites chambres annulaires successives.

Selon le cas typique, les ouvertures 111a, 111b, 111c réalisées dans la paroi cylindrique 111 du conteneur 110 sont disposées respectivement en regard des ouvertures 112a, 112b, 112c réalisées dans la paroi cylindrique 112 du conteneur 110. En d'autres termes, chaque ouverture 111a, 111b, 111c réalisée dans la paroi cylindrique interne 111 du conteneur 110 est positionnée en regard de d'une ouverture 112a, 112b, 112c correspondante réalisée dans la paroi cylindrique coaxiale 112 positionnée à l'extérieur de ladite paroi 111.

Par contre, les ouvertures 121a, 121b, 121c formées dans la paroi cylindrique 121 du couvercle 120 sont positionnées de manière décalée par rapport aux ouvertures 122a, 122b, 122c réalisées dans la paroi cylindrique coaxiale 122 du couvercle 120 située à l'extérieur de ladite paroi cylindrique 121, avec un décalage angulaire d'environ 60 degrés, de telle sorte que les ouvertures réalisées dans une paroi ne sont pas positionnées en regard des ouvertures formées dans l'autre paroi coaxiale successive du couvercle.

Ainsi, avec un tel agencement, lorsque le couvercle 120 est assemblé avec le conteneur 110, il est positionné par rapport à ce dernier de telle manière que les ouvertures d'une paire de parois cylindriques sont en regard l'une de l'autre pour mettre en communication deux chambres annulaires successives, les ouvertures de l'autre paire de parois cylindriques étant positionnées de manière décalée angulairement de sorte que les deux autres chambres annulaires successives sont isolées l'une de l'autre.

En outre, comme le montrent les figures 1 à 4, le couvercle 120 et le conteneur 110 sont pourvus de moyens de positionnement indexé.

Suivant le mode de réalisation représenté, ces moyens de positionnement indexé du couvercle 120 et du conteneur 110, comprennent d'une part, une ouverture 123a s'étendant sur un secteur angulaire de la paroi cylindrique 123 du couvercle 120 située à l'extérieur des autres parois cylindriques coaxiales et formant le bord périphérique externe du récipient, et d'autre part, un téton 113a s'étendant radialement en saillie de la paroi cylindrique 113 du conteneur 110 destinée à être positionnée contre la paroi cylindrique externe 123 du couvercle 120, ledit téton 113a étant apte à s'engager dans ladite ouverture 123a de la paroi externe 123 de couvercle 120 et à naviguer dans cette ouverture 123a lors de la rotation relative du couvercle 120 et du conteneur 110, de manière à venir se placer en butée contre les deux bords latéraux d'extrémité 123'a, 123''a de cette ouverture 123a.

Ici, l'ouverture 123a pratiquée dans la paroi externe 123 du couvercle 120 est réalisée par une encoche qui s'étend sur environ 70 degrés.

Les deux positions en butée du téton 113a dans l'ouverture 123a correspondent à deux positions relatives déterminées du couvercle 120 et du conteneur 110.

Une première position en butée du téton 113a contre le bord d'extrémité 123'a de ladite ouverture 123a, correspond ici à la mise en communication de la chambre d'admission de l'échantillon du produit 102 avec la chambre de fixation et de lecture du composant marqué 103, la chambre d'évacuation 104 étant alors isolée des autres chambres.

La deuxième position en butée du téton 113a contre l'autre bord d'extrémité 123''a de ladite ouverture 123 correspond ici à la mise en communication de la chambre de fixation et de lecture 103 avec la chambre d'évacuation 104 et à l'isolation de la chambre d'admission 102 des autres chambres.

Afin de faciliter la rotation relative du couvercle 120 et du conteneur 110, le couvercle 120 comporte un téton 123b s'étendant radialement en saillie de la face externe de la paroi cylindrique 123 située à l'extérieur des autres parois cylindriques, ledit téton 123b formant une prise ou un appui pour faire tourner le couvercle 120 autour de l'axe vertical 101 par rapport au conteneur 110.

Le conteneur 110 et le couvercle 120 sont pourvus chacun d'un orifice traversant central 105 isolé de la chambre annulaire immédiatement adjacente, ici la chambre d'admission 102, par une paroi cylindrique coaxiale 105a, 105b. Lorsque le couvercle est assemblé avec le conteneur les parois 105a et 105b du conteneur 110 et du couvercle 120 viennent se positionner l'une contre l'autre et le récipient fermé ainsi formé comporte un orifice central traversant, isolé par les deux parois 105a, 105b cylindriques positionnées l'une contre l'autre, de la chambre annulaire immédiatement adjacente 102.

Cet orifice central traversant 105 est destiné à s'enfiler sur un arbre vertical d'entraînement en rotation pour la mise en rotation dudit récipient. L'orifice 105 présente ici un diamètre d'environ 4mm.

Les ouvertures pratiquées dans les parois cylindriques coaxiales du conteneur 110 et du couvercle 105 pour la mise en communication des chambres annulaires du récipient présentent une largeur d'environ 5 mm.

Comme le montre plus particulièrement la figure 1, le couvercle 120 comporte dans son fond 120a un orifice 102a débouchant dans la chambre d'admission 102. Il est placé de manière adjacente à l'orifice traversant 105 puisque la chambre d'admission est immédiatement adjacente audit orifice traversant 105.

Bien entendu, on pourrait prévoir que cet orifice d'entrée dans la chambre d'admission soit réalisé selon une variante non représentée dans le fond 110a du conteneur 110.

Le conteneur 110 est réalisé en une matière transparente de manière à permettre la lecture des composants marqués fixés dans chaque chambre de fixation et de lecture, au travers de la paroi de fond dudit conteneur par exemple à l'aide d'une caméra CCD, par transmission et réflexion d'un rayonnement.

Le couvercle peut être alors opacifié, soit traité de façon à éviter des rayonnements parasites.

Avantageusement, le conteneur et le couvercle sont réalisés par moulage d'une matière plastique, les parois cylindriques coaxiales venant de formation avec ledit couvercle.

Toute matière plastique classiquement utilisée pour le « coating » de molécules peut être utilisée pour réaliser le dispositif selon l'invention. On utilisera par exemple du polystyrène, ou de préférence, un plastique de ZYLAR (Marque enregistrée), un tel plastique présentant une très forte capacité de fixation en terme de « coating ».

Selon une variante dudit dispositif de dosage, il peut être avantageusement prévu que la chambre de fixation et de lecture 103 soit divisée en une pluralité de secteurs angulaires sur lesquels sont fixés des récepteurs différents les uns des autres destinés chacun à la fixation et à la lecture d'un composant marqué différents. Selon cette variante, il peut être particulièrement avantageux de prévoir qu'un secteur angulaire de la chambre de fixation soit exempt de récepteurs fixés de manière à constituer un secteur blanc sur lequel on pourra ultérieurement réaliser une lecture pour déterminer le zéro d'initialisation du dispositif.

Il peut être également prévu selon une autre variante que le récipient comporte entre la chambre d'admission et la chambre d'évacuation plusieurs autres chambres de fixation et de lecture successives concentriques pour la fixation de composants marqués différents.

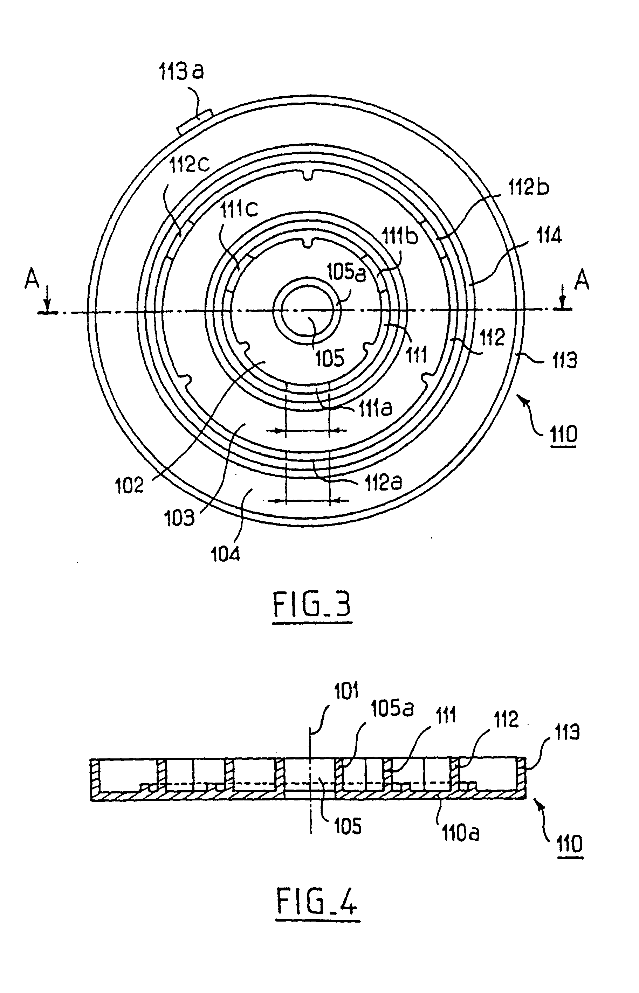

Le dispositif de dosage réalisé par l'assemblage du conteneur 110 et du couvercle 120 tels que représentés sur les figures 1 et 3, permet la mise en oeuvre d'un procédé de dosage qualitatif et/ou quantitatif d'au moins un composant particulier dans un échantillon de produit par marquage de fixation. Ce procédé va être décrit ci-après.

Selon ce procédé, on utilise au moins un dispositif du type décrit précédemment avec les deux éléments, conteneur et couvercle, tels que représentés sur les figures 1 et 3, assemblés pour former le récipient fermé, qui contient des récepteurs spécifiques du composant à doser fixés dans la chambre de fixation et de lecture.

Lors d'une première étape a), on place alors dans la chambre d'admission isolée des autres chambres annulaires dudit récipient, l'échantillon de produit contenant le composant à doser marqué.

Puis lors d'une étape b), on fait tourner le couvercle par rapport au conteneur de manière à mettre en communication la chambre d'admission avec la chambre de fixation et de lecture, la chambre d'évacuation étant isolée des autres chambres annulaires.

Puis lors d'une étape c), on entraîne en rotation le dispositif autour de son axe vertical de manière à disperser par centrifugation dans la chambre de fixation et de lecture l'échantillon de produit contenant le composant à doser marqué, ce dernier venant alors se lier par interaction forte aux récepteurs spécifiques fixés dans la chambre de fixation et de lecture.

Il est intéressant de souligner que la rotation du dispositif permet le transfert de l'échantillon de produit de la chambre d'admission dans la chambre de fixation et de lecture, mais aussi l'agitation de l'échantillon à l'intérieur de cette dernière pour permettre au composant marqué de se lier aux récepteurs fixés.

Lors d'une étape suivante d), on fait tourner le couvercle par rapport au conteneur de manière à mettre en communication la chambre de fixation et de lecture avec la chambre d'évacuation, puis lors d'une étape e) on entraîne en rotation le dispositif autour de son axe de manière à disperser par centrifugation le surplus d'échantillon dans la chambre d'évacuation.

Il convient de préciser qu'à cet effet, comme le montrent les figures 3 et 4, il est prévu dans le fond 110a du conteneur 110 du dispositif, une nervure circulaire 114 à proximité de la paroi cylindrique intérieure 112 formant la séparation entre la chambre de fixation et de lecture, et la chambre d'évacuation, cette nervure circulaire 114 formant un rebord anti-retour pour le surplus d'échantillon évacué par centrifugation dans ladite chambre d'évacuation, ou encore pour le liquide de rinçage récupéré dans cette chambre comme cela sera décrit ci-après.

Lors d'une étape f), on effectue plusieurs rinçages de l'intérieur du dispositif à l'aide d'un liquide de rinçage que l'on fait circuler par centrifugation dans les différentes chambres annulaires dudit dispositif en reproduisant les étapes b), c), d) et e) précédentes afin d'éliminer les autres composants dudit produit qui peuvent être accrochés par adsorption sur les parois internes du dispositif ou encore liés par interaction faible (telle que l'adsorption) aux récepteurs spécifiques fixés dans la chambre de fixation et de lecture.

Ainsi, on garde dans la chambre de fixation et de lecture après rinçage, seulement le composant marqué, lié par interaction forte aux récepteurs fixés dans ladite chambre.

On peut alors à l'étape f) détecter et doser au travers de la ou les paroi(s) dudit dispositif le composant marqué lié aux récepteurs fixés de manière à effectuer un dosage qualitatif et/ou quantitatif de ce composant marqué.

Cette détection peut être réalisée avantageusement selon le procédé conforme à l'invention à l'aide d'une caméra CCD. Pour cela, il faut que le marquage du composant à doser soit réalisé par voie physique ou chimique à l'aide par exemple de microsphères fluorescentes ou qui sont rendues fluorescentes.

La lecture des agents marqués fixés peut être effectuée selon des rayons de la chambre de fixation et de lecture.

Plus particulièrement, selon le procédé conforme à l'invention à l'aide d'une caméra CCD, on compte le nombre de composants marqués fixés dans chaque chambre de fixation et de lecture. Cela est possible en utilisant comme éléments de marquage susceptibles de marquer le composant à doser, des particules ou microsphères de préférence environ 100 fois plus grosses que les molécules recherchées, et conjuguées à l'anticorps ou l'antigène de révélation. Pour une meilleure résolution par la caméra CCD, on privilégie des particules dont le diamètre est de 2µm.

Ces microsphères sont telles qu'elles réfléchissent tout ou partie du rayonnement qu'elles reçoivent. Elles peuvent être constituées de latex ou tout autre matériau permettant leur détection et leur comptage.

Ainsi, la caméra CCD capte un nombre déterminé d'événements correspondant à un nombre absolu de composants à doser fixés.

La caméra CCD est raccordée à un software qui donne en sortie un signal numérique de détection. Un tel système visionique fournit ainsi un comptage en temps réel de quelques unités à 100 000 microsphères par mm², avec soustraction du bruit de fond à l'aide d'une surface de référence et dialgorithmes appropriés. Il possède également un pouvoir de discrimination dans la mesure où il peut reconnaître et écarter les images hétérogènes susceptibles de fausser l'analyse des données.

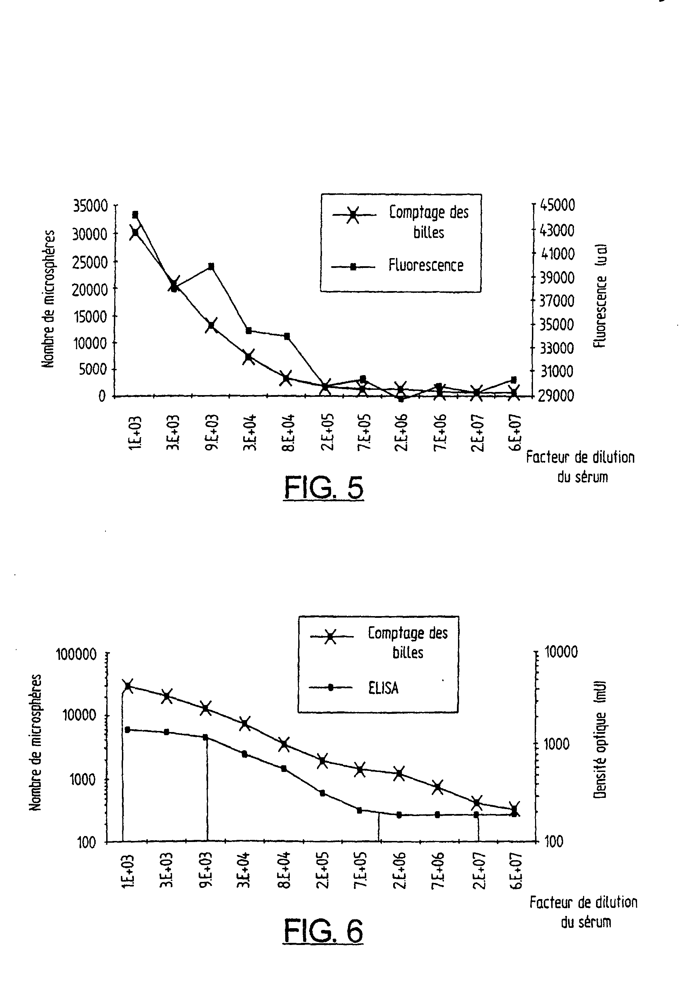

Les figures 5 et 6 montrent des courbes de résultats obtenus avec la méthode de détection par comptage précitée selon l'invention et des méthodes de détection plus classiques du type par fluorescence ou ELISA, pour un composant à doser donné dans un sérum donné.

Les courbes des figures 5 et 6 permettent de vérifier d'une part qu'il y a bien une corrélation entre le comptage des microsphères et la concentration du composant à doser, et d'autre part que la méthode de comptages des microsphères selon l'invention est plus précise que les méthodes de détection dites classiques. En particulier, le dosage par comptage de microsphères offre une dynamique de plus de 4Log contre 2Log pour la méthode ELISA. Ceci est particulièrement avantageux puisqu'on augmente ainsi considérablement la limite de détection. Les résultats obtenus avec de fortes dilutions de l'échantillon montrent ainsi que la méthode présente une sensibilité 2Log supérieure à celle de l'ELISA. Les résultats obtenus sur de faibles dilutions de l'échantillon montrent également que la méthode permet encore de réaliser des dosages à des concentrations d'analyte correspondant à un seuil de saturation en ELISA. Ceci peut donc avantageusement permettre d'éviter ou de diminuer d'éventuels effets de dilution sur certains composants d'échantillon à tester.

Selon le procédé précité, le marquage de chaque composant particulier de l'échantillon peut être réalisé à l'extérieur avant l'introduction dudit échantillon dans ladite chambre d'admission du dispositif.

En variante, le marquage de chaque composant particulier à doser de l'échantillon de produit peut être réalisé directement dans la chambre d'admission et de marquage, en introduisant dans une première étape un récepteur marqué spécifique de chaque composant à doser, sous une forme sèche non fixée, puis dans une deuxième étape en introduisant l'échantillon de produit dans ladite chambre d'admission isolée, de sorte que le récepteur marqué vienne se lier par interaction forte aux composant correspondant contenu dans ledit échantillon de produit.

Bien entendu, ce procédé décrit à l'aide d'un dispositif et tel que représenté sur les figures 1 à 4, peut être réalisé à l'aide d'une pluralité dé dispositifs de ce type, pour doser simultanément un même composant particulier dans une pluralité d'échantillons différents ou encore doser différents composants particuliers dans un même échantillon de produit.

A cet effet, il est prévu avantageusement selon l'invention un appareil pour la mise en oeuvre de ce procédé utilisant une pluralité de dispositifs du type de celui représenté sur les figures 1 à 4, qui comprend un arbre vertical d'entraînement en rotation sur lequel sont enfilés lesdits dispositifs de dosage, des moyens pour maintenir à distance les uns des autres lesdits dispositifs, des moyens pour entraîner en rotation dans les deux sens ledit arbre vertical d'entraînement de façon à réaliser la centrifugation par rotation desdits dispositifs, des moyens d'injection d'échantillons de produit et du liquide de rinçage dans les chambres d'admission des dispositifs enfilés sur l'arbre d'entraînement et des moyens pour faire tourner les couvercles relativement aux conteneurs des dispositifs de manière à mettre en communication ou à isoler les différentes chambres annulaires successives desdits dispositifs, et un moyen de lecture des agents marqués fixés.

L'invention n'est nullement limitée aux modes de réalisation décrits et représentés.

## Revendications

1. Dispositif pour effectuer un dosage qualitatif et/ou quantitatif d'au moins un composant particulier dans un échantillon de produit par marquage et fixation, dispositif comprenant un conteneur (110) et un couvercle (120) assemblés pour former un récipient fermé, **caractérisé en ce que** ledit récipient fermé présente un axe vertical (101), **en ce que** le conteneur (110) et le couvercle (120) portent des parois cylindriques coaxiales (111,112,113 ; 121,122,123) qui lors de l'assemblage de ces derniers se positionnent par paire l'une contre l'autre en délimitant au moins trois chambres annulaires concentriques (102,103,104) à l'intérieur du récipient, à savoir depuis l'axe une chambre d'admission (102) destinée à recevoir l'échantillon et le cas échéant permettant le marquage du composant, une chambre de fixation et de lecture dudit composant marqué (103) et une chambre d'évacuation (104), **en ce que** les parois cylindriques coaxiales (111,112 ; 121,122) formant des séparations entre les chambres annulaires successives (102,103,104) comprennent chacune au moins une ouverture (111a,112a ; 121a,122a), et **en ce que** le couvercle (120) et le conteneur (110) assemblés sont aptes à tourner l'un par rapport à l'autre autour de l'axe vertical (101) et les ouvertures des parois cylindriques coaxiales du conteneur (110) et du couvercle (120) sont placées à des positions angulaires déterminées, de manière que par déplacement l'une par rapport à l'autre des parois cylindriques (111, 112 ; 121, 122)de chaque paire, les ouvertures de chaque paire de parois sont aptes à être positionnées en regard l'une de l'autre ou de manière décalée angulairement, pour mettre en communication ou isoler l'une de l'autre les chambres annulaires successives.

2. Dispositif selon la revendication 1, **caractérisé en ce que** lesdites ouvertures prévues dans les parois cylindriques coaxiales du conteneur (110) et du couvercle (120) sont positionnées de telle manière que lorsque les ouvertures d'une paire de parois cylindriques sont en regard l'une de l'autre pour mettre en communication deux chambres annulaires successives, les ouvertures des autres paires de parois cylindriques sont positionnées de manière décalée angulairement de sorte que les autres chambres annulaires sont isolées.

3. Dispositif selon l'une des revendications 1 ou 2, **caractérisé en ce qu'**il comporte sur le couvercle (120) et sur le conteneur (110) des moyens de positionnement indexé de ces derniers.

4. Dispositif selon la revendication 3, **caractérisé en ce que** lesdits moyens de positionnement indexé du couvercle (120) et du conteneur (110), comprennent d'une part une ouverture (123a) s'étendant sur un secteur angulaire de la paroi cylindrique (123) du couvercle (120) située à l'extérieur et formant le bord périphérique externe du récipient et d'autre part un téton (113a) s'étendant radialement en saillie de la paroi cylindrique (113) du conteneur (110) positionnée contre ladite paroi cylindrique externe (123) du couvercle (120), ledit téton (113a) étant destiné à s'engager dans ladite ouverture (123a) de la paroi cylindrique externe (123) du couvercle et à naviguer dans cette ouverture lors de la rotation relative du couvercle et du conteneur, de manière à venir se placer en butée contre les deux bords latéraux d'extrémité (123'a, 123''a) de cette ouverture (123a), les deux positionnements en butée du téton (113a) dans l'ouverture (123a) correspondant à deux positions relatives déterminées du couvercle (120) et du conteneur(110).

5. Dispositif selon la revendication 4, **caractérisé en ce qu'**une première position en butée du téton (113a) dans ladite ouverture (123a) correspond à la mise en communication de la chambre d'admission de l'échantillon (102) avec la chambre de fixation et de lecture du composant marqué (103), la chambre d'évacuation (104) étant isolée des autres chambres, et une deuxième position en butée du téton (113a) dans ladite ouverture (123a) correspond à la mise en communication de la chambre de fixation et de lecture (103) avec la chambre d'évacuation (104) et à l'isolation de la chambre d'admission (102).

6. Dispositif selon l'une quelconque des revendications précédentes, **caractérisé en ce que** le couvercle (120) comporte un téton (123b) sur la face externe d'une de ses parois cylindriques coaxiales (123) située à l'extérieur des autres parois cylindriques de façon à former le bord périphérique externe du récipient, ledit téton (123b) formant une prise ou un appui pour faire tourner le couvercle (120) autour de l'axe vertical (101) par rapport au conteneur (110).

7. Dispositif selon l'une quelconque des revendications précédentes, **caractérisé en ce que** le récipient comporte un orifice central traversant (105) isolé de la chambre annulaire immédiatement adjacente (102) et destiné à s'enfiler sur un arbre vertical d'entraînement en rotation pour la mise en rotation dudit récipient.

8. Dispositif selon l'une quelconque des revendications précédentes, **caractérisé en ce qu'**il comprend dans le fond (120a) du couvercle (120) ou dans le fond (110a) du conteneur (110), un orifice d'entrée (102a) dans la chambre d'admission (102).

9. Dispositif selon l'une quelconque des revendications 1 à 8, **caractérisé en ce que** le récipient comporte entre la chambre d'admission et la chambre d'évacuation plusieurs chambres de fixation et de lecture concentriques.

10. Dispositif selon l'une quelconque des revendications précédentes, **caractérisé en ce que** lesdites parois cylindriques coaxiales (111,112 ; 121,122) formant des séparations entre les chambres annulaires successives (102,103,104) comprennent chacune trois ouvertures (111a,111b,111c,112a,112b,112c ; 121a,121b,121c,122a,122b,122c) réparties régulièrement sur leur circonférence.

11. Dispositif selon l'une quelconque des revendications précédentes, **caractérisé en ce que** le récipient présente la forme générale d'un disque.

12. Dispositif selon l'une quelconque des revendications précédentes, **caractérisé en ce que** le conteneur (110) est réalisé en un matériau transparent de manière à permettre une lecture des composants marqués fixés dans la chambre de fixation et de lecture au travers des parois dudit récipient.

13. Dispositif selon l'une quelconque des revendications 1 à 12, **caractérisé en ce qu'**il est réalisé en matière plastique.

14. Dispositif selon l'une des revendications 1 à 13, **caractérisé en ce qu'**il comporte dans la chambre de fixation (103) au moins un récepteur du composant à doser, ledit récepteur étant fixé sur au moins l'une des parois de la chambre.

15. Dispositif selon l'une des revendications 1 à 14, **caractérisé en ce que** ledit récepteur est fixé sur la paroi de fond (110a) du conteneur (110).

16. Dispositif selon l'une des revendications 1 à 15, **caractérisé en ce que** la chambre d'admission (102) comporte un élément de marquage susceptible de marquer le composant à doser.

17. Dispositif selon l'une des revendications 1 à 16, **caractérisé en ce que** le récepteur fixé est choisi parmi :
- les anticorps, les antigènes, les séquences d'acides nucléiques complémentaires, les récepteurs vrais pour doser respectivement des composants qui sont :
- les antigènes, les anticorps, et les séquences d'acides nucléiques, les ligands des récepteurs.

18. Dispositif selon l'une quelconque des revendications 1 à 17, **caractérisé en ce que** la chambre de fixation et de lecture (103) est divisée en une pluralité de secteurs angulaires sur lesquels sont fixés des récepteurs différents les uns des autres destinés chacun à la fixation et à la lecture d'un composant marqué différent.

19. Dispositif selon la revendication 18, **caractérisé en ce qu'**un secteur angulaire de la chambre de fixation est laissé exempt de récepteurs de manière à constituer un secteur blanc destiné à la réalisation d'une lecture d'initialisation dudit dispositif.

20. Dispositif selon l'une des revendications 16 ou 17, **caractérisé en ce que** le marquage est effectué par des moyens physiques ou chimiques.

21. Dispositif selon la revendication 20, **caractérisé en ce que** le marquage est effectué à l'aide de particules et/ou d'éléments fluorescents ou qui peuvent être rendus fluorescents.

22. Dispositif selon la revendication 21, **caractérisé en ce que** les particules présentent un diamètre suffisant, supérieur ou égal à environ 100 fois le diamètre du ou des composant(s) à doser et possèdent des propriétés optiques permettant leur détection par comptage.

23. Dispositif selon la revendication 21, **caractérisé en ce que** les particules ont un diamètre de 2µm.

24. Dispositif selon l'une des revendications 16 à 23, **caractérisé en ce que** l'élément de marquage est constitué par un anticorps marqué ou un antigène marqué, ou une séquence d'acide nucléique marquée ou tout élément de récepteur qui peut être marqué.

25. Dispositif selon l'une des revendications précédentes, **caractérisé en ce que** le couvercle est traité de façon à éviter des rayonnements parasites et **en ce que** la lecture est effectuée à l'aide d'une caméra CCD.

26. Dispositif selon la revendication 25, **caractérisé en ce que** la caméra CCD est apte à compter de manière discrétionnaire par émission/réception d'un signal lumineux, le nombre des composants marqués fixés dans chaque chambre de fixation et de lecture, de façon à obtenir un signal de détection numérique.

27. Procédé de dosage qualitatif et/ou quantitatif d'au moins un composant particulier dans un échantillon de produit par marquage et fixation, **caractérisé en ce qu'**on utilise au moins un dispositif selon l'une quelconque des revendications 1 à 26 qui contient des récepteurs spécifiques du composant à doser fixés dans chaque chambre de fixation et de lecture et dans lequel,
a) on place dans la chambre d'admission isolée des autres chambres annulaires, l'échantillon de produit contenant le composant marqué,
b) on fait tourner le couvercle par rapport au conteneur de manière à mettre en communication la chambre d'admission avec la chambre de fixation et de lecture, la chambre d'évacuation étant isolée des autres chambres annulaires,
c) on entraîne en rotation le dispositif autour de son axe vertical de manière à disperser par centrifugation dans chaque chambre de fixation et de lecture l'échantillon de produit contenant le composant marqué, ce dernier venant alors se lier par interaction forte aux récepteurs spécifiques fixés dans chaque chambre de fixation et de lecture,
d) on fait tourner le couvercle par rapport au conteneur de manière à mettre en communication la chambre de fixation et de lecture avec la chambre d'évacuation,
e) on entraîne en rotation le dispositif autour de son axe de manière à disperser par centrifugation le surplus d'échantillon dans la chambre d'évacuation,
f) on rince l'intérieur du dispositif à l'aide d'un liquide de rinçage que l'on fait circuler par centrifugation dans les différentes chambres annulaires dudit dispositif en reproduisant les étapes b), c), d) et e) précédentes de manière à garder dans la chambre de fixation et de lecture seulement le composant marqué lié par interaction forte aux récepteurs fixés,
g) on détecte et on dose au travers de la ou les paroi(s) dudit dispositif ledit composant marqué par un moyen approprié.

28. Procédé selon la revendication 27, **caractérisé en ce que** le marquage de chaque composant particulier à doser de l'échantillon de produit, est réalisé à l'extérieur avant l'introduction de celui-ci dans la chambre d'admission du dispositif.

29. Procédé selon la revendication 27, **caractérisé en ce que** le marquage de chaque composant à doser de l'échantillon de produit, est réalisé dans la chambre d'admission et de marquage, en introduisant préalablement à l'échantillon de produit un élément de marquage spécifique de chaque composant à doser, de préférence sous forme sèche non fixée, puis en introduisant l'échantillon de produit dans ladite chambre d'admission isolée de sorte que l'élément de marquage vient se lier par interaction forte au composant correspondant contenu dans ledit échantillon de produit.

30. Procédé selon l'une des revendications 27 à 29, **caractérisé en ce que** l'opération de lecture permettant la détection du composant marqué fixé dans la chambre de fixation et de lecture est réalisée à l'aide d'une caméra CCD.

31. Procédé selon la revendication 30, **caractérisé en ce que** la caméra CCD réalise un comptage discrétionnaire par émission/réception d'un signal lumineux des composants marqués fixés dans chaque chambre de fixation et de lecture, de façon à obtenir un signal de détection numérique.

32. Procédé selon l'une des revendications 27 à 31, **caractérisé en ce que** la lecture des agents marqués fixés est effectuée selon des rayons de la chambre de fixation et de lecture.

33. Appareil pour la mise en oeuvre du procédé selon l'une des revendications 27 à 32, **caractérisé en ce qu'**il comprend un arbre vertical d'entraînement en rotation sur lequel sont enfilés des dispositifs selon l'une des revendications 1 à 26, des moyens pour maintenir à distance les uns des autres lesdits dispositifs, des moyens pour entraîner en rotation dans les deux sens ledit arbre vertical d'entraînement, des moyens d'injection d'échantillons de produit et du liquide de rinçage dans les chambres d'admission desdits dispositifs enfilés sur l'arbre d'entraînement, et des moyens pour faire tourner les couvercles des dispositifs relativement aux conteneurs de manière mettre en communication ou à isoler les différentes chambres annulaires successives de ces derniers et un moyen de lecture des agents marqués fixés.

## Patentansprüche

1. Vorrichtung zum Bewirken einer qualitativen und/oder quantitativen Bestimmung mindestens eines speziellen Bestandteils bzw. einer speziellen Verbindung in einer Produktprobe durch Markierung und Fixierung, wobei die Vorrichtung einen Container (110) und einen Deckel (120) aufweist, die zusammengesetzt sind, um einen geschlossenen Aufnahmebehälter zu bilden, **dadurch gekennzeichnet, daß** der genannte, geschlossene Behälter eine vertikale Achse (101) aufweist, daß der Container (110) und der Deckel (120) zylindrische, koaxiale Wände (111, 112, 113; 121, 122, 123) tragen, die sich während des Zusammensetzens dieser letztgenannten paarweise gegeneinander positionieren, wobei sie mindestens drei ringförmige, konzentrische Kammern (102, 103, 104) im Inneren des Behälters umgrenzen, und zwar, von der Achse aus, eine Eintrittekammer (102), die zur Aufnahme der Probe bestimmt ist und, je nachdem, die Markierung der Verbindung gestattet, eine Fixierungs- und Ablesungskammer (103) für die genannte, markierte Verbindung, und eine Entleerungskammer (104), daß die zylindrischen, koaxialen Wände (111, 112; 121, 122) zwischen den aufeinanderfolgenden, ringförmigen Kammern (102, 103, 124) Abtrennungen bilden, von denen jede mindestens eine Öffnung (111a, 112a; 121a, 122a) aufweist, und daß der Deckel (120) und der Container (110) im zusammengesetzten Zustand geeignet sind, sich gegeneinander um die vertikale Achse (101) zu drehen, und die Öffnungen der zylindrischen, koaxialen wände des Containers (110) und des Deckels (120) an bestimmten Winkellagen derart angeordnet sind, daß durch die Versetzung der einen zylindrischen Wand (111, 112; 121, 122) eines jeden Paares bezüglich der anderen die Öffnungen eines jeden Paares von Wänden dazu geeignet sind, einander gegenüberliegend oder in winklig versetzter Weise positioniert zu sein, um die eine der ringförmigen, aufeinanderfolgenden Kammern gegenüber der anderen in Verbindung zu setzen oder zu isolieren.

2. Vorrichtung nach Anspruch 1, **dadurch gekennzeichnet, daß** die genannten Öffnungen, die in den zylindrischen, koaxialen Wänden des Containers (110) und des Deckels (120) vorgesehen sind, derart positioniert sind, daß dann, wenn die Öffnungen eines Paares von zylindrischen Wänden einander zugewandt sind, um zwei aufeinanderfolgende, ringförmige Kammern in Verbindung zu setzen, die Öffnungen der anderen Paare von zylindrischen Wänden in winklig versetzter Weise derart angeordnet sind, daß die anderen, ringförmigen Kammern isoliert sind.

3. Vorrichtung nach einem der Ansprüche 1 oder 2, **dadurch gekennzeichnet, daß** sie auf dem Deckel (120) und auf dem Container (110) Mittel zur indexierten Positionierung dieser letztgenannten aufweisen.

4. Vorrichtung nach Anspruch 3, **dadurch gekennzeichnet, daß** die genannten Mittel zur indexierten Positionierung des Deckels (120) und des Containers (110) einerseits eine Öffnung (123a), die sich auf einem Winkelsektor der zylindrischen Wand (123) des Deckels (120) erstreckt, die auf der Außenseite liegt und den äußeren Umfangsrand des Behälters bildet, und andererseits eine Kuppe (113a) aufweisen, die sich radial, von der zylindrischen Wand (113) des Containers (110), die gegen die genannte zylindrische Außenwand (123) des Deckels (120) angeordnet ist, vorspringend, erstreckt, wobei die genannte Kuppe (113a) dazu bestimmt ist, in die genannte Öffnung (123a) der zylindrischen, äußeren Wand (123) des Deckels einzugreifen und in dieser Öffnung während der relativen Drehung des Deckels und des Behälters derart zu steuern, daß sie zum Anschlag gegen die beiden seitlichen Endwände (123'a, 123''a) dieser Öffnung (123a) gelangt, wobei die beiden Anschlagspositionierungen der Kuppe (113a) in der Öffnung (123a) den beiden bestimmten Relativlagen des Deckels (120) und des Containers (110) entspricht.

5. Vorrichtung nach Anspruch 4, **dadurch gekennzeichnet, daß** eine erste Anschlagsposition der Kuppe (113a) in der genannten Öffnung (123a) der Herstellung der Verbindung der Probe-Eintrittskammer (102) mit der Fixierungs- und Ablesungskammer (103) der markierten Verbindung entspricht, wobei die Entleerungskammer (104) gegenüber den anderen Kammern isoliert ist, und eine zweite Anschlagposition der Kuppe (113a) in der genannten Öffnung (123a) der Herstellung der Verbindung der Fixierungs- und Ablesungskammer (103) mit der Entleerungskammer (104) und der Isolierung der Eintrittskammer (102) entspricht.

6. Vorrichtung nach irgendeinem der vorhergehenden Ansprüche, **dadurch gekennzeichnet, daß** der Deckel (120) eine Kuppe (123b) auf der Außenseite einer seiner zylindrischen, koaxialen Wände(123) aufweist, die auf der Außenseite der anderen zylindrischen Wände derart gelegen ist, daß sie den Außenumfangsrand des Behälters bildet, wobei die genannte Kuppe (123b) eine Aufnahme oder einen Anschlag bildet, um die Drehung des Deckels (120) rund um die vertikale Achse (101) bezüglich dem Container (110) zuzulassen.

7. Vorrichtung nach irgendeinem der vorhergehenden Ansprüche, **dadurch gekennzeichnet, daß** der Behälter eine mittige Durchgangsöffnung (105) aufweist, die gegenüber der unmittelbar benachbarten, ringförmigen Kammer (102) isoliert ist und dazu bestimmt ist, sich auf einer vertikalen Drehantriebswelle aufzureihen, um den genannten Behälter in Drehung zu versetzen.

8. Vorrichtung nach irgendeinem der vorhergehenden Ansprüche, **dadurch gekennzeichnet, daß** sie im Boden (120a) des Deckels (120) oder im Boden (110a) des Containers (110) eine Einlaßöffnung (102a) in die Eintrittskammer (102) aufweist.

9. Vorrichtung nach irgendeinem der Ansprüche 1 bis 8, **dadurch gekennzeichnet, daß** der Behälter zwischen der Eintrittskammer und der Entleerungskammer mehrere konzentrische Fixierungs- und Ablesungskammern aufweist.

10. Vorrichtung nach irgendeinem der vorhergehenden Ansprüche, **dadurch gekennzeichnet, daß** die genannten, zylindrischen, koaxialen Wände (111, 112; 121, 122), die die Abtrennungen zwischen den ringförmigen, aufeinanderfolgenden Kammern (102, 103, 104) bilden, jeweils drei Öffnungen (111a, 111b, 111c, 112a, 112b, 112c; 121a, 121b, 121c, 122a, 122b, 122c) aufweisen, die gleichmäßig über ihren Umfang verteilt sind.

11. Vorrichtung nach irgendeinem der vorhergehenden Ansprüche, **dadurch gekennzeichnet, daß** der Behälter die allgemeine Form einer Scheibe aufweist.

12. Vorrichtung nach irgendeinem der vorhergehenden Ansprüche, **dadurch gekennzeichnet, daß** der Container (110) aus einem transparenten Material derart hergestellt ist, daß er eine Ablesung der markierten Verbindung durch die Wände des genannten Behälters hindurch gestattet, die in der Fixierungs- und Ablesungskammer festgelegt sind.

13. Vorrichtung nach irgendeinem der Ansprüche 1 bis 12, **dadurch gekennzeichnet, daß** sie aus Kunststoff hergestellt ist.

14. Vorrichtung nach einem der Ansprüche 1 bis 13, **dadurch gekennzeichnet, daß** sie in der Fixierungskammer (103) mindestens einen Rezeptor für die zu bestimmende Verbindung aufweist, wobei der genannte Rezeptor auf mindestens einer der Wände der Kammer fixiere ist.

15. Vorrichtung nach einem der Ansprüche 1 bis 14, **dadurch gekennzeichnet, daß** der genannte Rezeptor auf der Bodenwand (110a) des Containers (110) fixiert ist.

16. Vorrichtung nach einem der Ansprüche 1 bis 15, **dadurch gekennzeichnet, daß** die Eintrittskammer (102) ein Markierungselement aufweist, das in der Lage ist, die zu bestimmende Verbindung zu markieren.

17. Vorrichtung nach einem der Ansprüche 1 bis 16, **dadurch gekennzeichnet, daß** der fixierte Rezeptor ausgewählt ist unter:
- den Antikörpern. Antigenen, komplementären Nukleinsäuresequenzen, und echten Rezeptoren zu bestimmender Verbindungen, die folgende sind:
- die Antigene. Antikörper und Nukleinsäuresequenzen, die Liganden der Rezeptoren.

18. Vorrichtung nach irgendeinem der Ansprüche 1 bis 17, **dadurch gekennzeichnet, daß** die Fixierungs- und Ablesungskammer (103) in eine Vielzahl von Winkelsektoren unterteilt ist, auf denen verschiedene Rezeptoren fixiert sind, von denen jeder zur Fixierung und Ablesung einer Komponente bestimmt ist, die unterschiedlich markiert ist.

19. Vorrichtung nach Anspruch 18, **dadurch gekennzeichnet, daß** ein Winkelsektor der Fixierungskammer derart frei von Rezeptoren belassen ist, daß er einen weißen Sektor bildet, der zur Durchführung einer Anfangsablesung der genannten Vorrichtung bestimmt ist.

20. Vorrichtung nach einem der Ansprüche 16 oder 17, **dadurch gekennzeichnet, daß** die Markierung durch physikalische oder chemische Wirkstoffe bewirkt ist.

21. Vorrichtung nach Anspruch 20, **dadurch gekennzeichnet, daß** die Markierung mit Hilfe von fluoreszierenden Partikeln und/oder Elementen oder solcher Partikel und/oder Elemente bewirkt wird, die fluoreszierend gemacht werden können.

22. Vorrichtung nach Anspruch 21, **dadurch gekennzeichnet, daß** die Partikel einen ausreichenden Durchmesser aufweisen, größer oder gleich dem etwa 100-fachen des Durchmessers des oder der zu bestimmenden Verbindung(en), und optische Eigenschaften besitzen, die ihre Erfassung durch Zählung gestatten.

23. Vorrichtung nach Anspruch 21, **dadurch gekennzeichnet, daß** die Partikel einen Durchmesser von 2 µm aufweisen.

24. Vorrichtung nach einem der Ansprüche 16 bis 23, **dadurch gekennzeichnet, daß** das Markierungselement von einem markierten Antikörper oder einem markierten Antigen oder einer markierten Nukleinsäuresequenz oder jedem Rezeptorelement gebildet ist, das markiert werden kann.

25. Vorrichtung nach einem der vorausgehenden Ansprüche, **dadurch gekennzeichnet, daß** der Deckel derart behandelt ist, daß Parasitenstrahlen verhindert werden, und daß die Ablesung mit Hilfe einer CCD-Kamera bewirkt wird.

26. Vorrichtung nach Anspruch 25, **dadurch gekennzeichnet, daß** die CCD-Kamera dazu geeignet ist, in diskreter Weise durch Senden/Empfangen eines Leuchtsignals die Anzahl der markierten Verbindungen zu zählen, die in jeder Fixierungsund Ablesungskammer fixiert sind, um auf diese Weise ein numerisches Erfassungssignal zu erhalten.

27. Verfahren zur qualitativen und/oder quantitativen Bestimmung mindestens eines speziellen Bestandteils bzw. einer speziellen Verbindung in einer Produktprobe durch Markierung und Fixierung, **dadurch gekennzeichnet, daß** man mindestens eine Vorrichtung nach irgendeinem der Ansprüche 1 bis 26 verwendet, die spezielle Rezeptoren der zu bestimmenden Verbindung enthält, die in jeder Fixierungs- und Ablesungskammer fixiert sind, und in welchem
a) man in der Eintrittskammer, die von den anderen ringförmigen Kammern isoliert ist, die Produktprobe einsetzt, welche die markierten Verbindung enthält,
b) man den Deckel bezüglich des Containers sich derart drehen läßt, daß man die Eintrittskammer mit der Fixierungs- und Ablesungskammer in Verbindung setzt, wobei die Entleerungskammer gegenüber den anderen ringförmigen Kammern isoliert ist,
c) man die Vorrichtung zur Drehung um ihre vertikale Achse derart antreibt, daß man durch Fliehkraft in jeder Fixierungs- und Ablesungskammer die Produktprobe verteilt, welche die markierte Verbindung enthält, wobei dieses letztgenannte sich dann durch kräftige Wechselwirkung mit den spezifischen Rezeptoren verbindet, die in jeder Fixierungs- und Ablesungskammer fixiert sind,
d) man den Deckel bezüglich des Containers derart drehen läßt, daß man die Fixierungs- und Ablesungskammer mit der Entleerungskammer in Verbindung setzt,
e) man die Vorrichtung zur Drehung um ihre Achse derart antreibt, daß man durch Fliehkraft den überschüssigen Teil der Probe in die Entleerungskammer verteilt,
f) man das Innere der Vorrichtung mit Hilfe einer Spülflüssigkeit spült, die man durch Fliehkraft in den verschiedenen ringförmigen Kammern der genannten Vorrichtung umlaufen läßt. indem man die vorausgehenden Schritte b), c), d) und e) reproduziert, um auf diese Weise in der Fixierungs- und Ablesungskammer alleine die markierte Verbindung zu bewahren, die durch starke Wechselwirkung mit den fixierten Rezeptoren verbunden ist, und
g) man durch die Wand oder Wände der genannten Vorrichtung die genannte Verbindung erfaßt und bestimmt, die durch ein geeignetes Mittel markiert ist.

28. Verfahren nach Anspruch 27, **dadurch gekennzeichnet, daß** die Markierung einer jeden speziellen, zu bestimmenden Verbindung der Produktprobe außen vor der Einbringung dieser in die Eintrittskammer der Vorrichtung durchgeführt wird.

29. Verfahren nach Anspruch 27, **dadurch gekennzeichnet, daß** die Markierung einer jeden, zu bestimmenden Verbindung der Produktprobe in der Eintritts- und Markierungskammer dadurch durchgeführt wird, daß man vorab der Produktprobe ein spezifisches Markierungselement für jede zu bestimmende Verbindung zuführt, bevorzugt in trockener, nicht fixierter Form, und dann die Produktprobe in die isolierte Eintrittskammer derart einbringt, daß das Markierungselement sich durch kräftige Wechselwirkung mit der entsprechenden Verbindung verbindet, die in der genannten Produktprobe enthalten ist.

30. Verfahren nach einem der Ansprüche 27 bis 29, **dadurch gekennzeichnet, daß** der Ablesungsvorgang des Erfassens der markierten, fixierten Verbindung in der Fixierungs- und Ablesungskammer mit Hilfe einer CCD-Kamera durchgeführt wird.

31. Verfahren nach Anspruch 30, **dadurch gekennzeichnet, daß** die CCD-Kamera eine diskrete Zählung durch Senden/Empfangen eines Leuchtsignals der markierten, in jeder Fixierungs- und Ablesungskammer fixierten Verbindung derart durchführt, daß ein numerisches Erfassungssignal erhalten wird.

32. Verfahren nach einem der Ansprüche 27 bis 31, **dadurch gekennzeichnet, daß** die Ablesung der fixierten, markierten Wirkstoffe entsprechend der Strahlen der Fixierungs- und Ablesungskammer bewirkt wird.

33. Gerät für die Durchführung des Verfahrens nach einem der Ansprüche 27 bis 32, **dadurch gekennzeichnet, daß** es eine vertikale Drehantriebswelle aufweist, auf der Vorrichtungen nach einem der Ansprüche 1 bis 26 aufgereiht sind, Mittel, um den Abstand der einen genannten Vorrichtungen von den anderen aufrecht zu erhalten, Mittel, um die genannte, vertikale Antriebswelle zur Drehung in den beiden Richtungen anzutreiben, Mittel zum Einspritzen von Produktproben und Spülflüssigkeit in die Eintrittskammern der genannten Vorrichtungen, die auf der Antriebswelle aufgereiht sind, und Mittel, um die Deckel der Vorrichtungen relativ zu den Containern drehen zu lassen, so daß die verschiedenen ringförmigen, aufeinanderfolgenden Kammern dieser letztgenannten in Verbindung gesetzt oder isoliert werden, und ein Ablesungsmittel der markierten, fixierten Wirkstoffe.

## Claims

1. Device for the qualitative and/or quantitative dosing of at least one particular component in a product sample by labelling and fixing, device comprising a container (110) and a cover (120) which are assembled to form a closed receptacle, **characterized in that** said closed receptacle has a vertical axis (101), **in that** the container (110) and the cover (120) carry coaxial cylindrical walls (111, 112, 113; 121, 122, 123) which, while the container and cover are being assembled, position themselves pairwise one against the other thereby delimiting at least three concentric annular chambers (102, 103, 104) inside the receptacle, namely from the axis an inlet chamber (102) intended for receiving the sample and as appropriate allowing the labelling of the component, a chamber for fixing and reading said labelled component (103) and a discharge chamber (104), **in that** the coaxial cylindrical walls (111, 112; 121, 122) forming separations between the successive annular chambers (102, 103, 104) each comprise at least one opening (111a, 112a; 121a, 122a), and **in that** the assembled cover (120) and container (110) are able to turn with respect to one another about the vertical axis (101) and the openings of the coaxial cylindrical walls of the container (110) and of the cover (120) are placed at determined angular positions, in such a way that by displacement of one with respect to the other of the cylindrical walls (111, 112; 121, 122) of each pair, the openings of each pair of walls are able to be positioned opposite one another or in an angularly offset manner, so as to put into communication or isolate from one another the successive annular chambers.

2. Device according to Claim 1, **characterized in that** said openings provided in the coaxial cylindrical walls of the container (110) and of the cover (120) are positioned in such a way that when the openings of a pair of cylindrical walls are opposite one another so as to put into communication two successive annular chambers, the openings of the other pairs of cylindrical walls are positioned in an angularly offset manner so that the other annular chambers are isolated.

3. Device according to either of Claims 1 and 2, **characterized in that** on the cover (120) and on the container (110) it comprises means of indexed positioning of these latter.

4. Device according to Claim 3, **characterized in that** said means of indexed positioning of the cover (120) and of the container (110), comprise on the one hand an opening (123a) extending over an angular sector of the cylindrical wall (123) of the cover (120) which is situated on the outside and forms the external peripheral edge of the receptacle and on the other hand a nipple (113a) extending radially while projecting from that cylindrical wall (113) of the container (110) which is positioned against said external cylindrical wall (123) of the cover (120), said nipple (113a) being intended for engaging in said opening (123a) of the external cylindrical wall (123) of the cover and for navigating in this opening during relative rotation of the cover and of the container, in such a way as to come into abutment against the two end lateral edges (123'a, 123''a) of this opening (123a), the two abutting positionings of the nipple (113a) in the opening (123a) corresponding to two determined relative positions of the cover (120) and of the container (110).

5. Device according to Claim 4, **characterized in that** a first abutting position of the nipple (113a) in said opening (123a) corresponds to the putting of the sample inlet chamber(102) into communication with the chamber for fixing and reading the labelled component (103), the discharge chamber (104) being isolated from the other chambers, and a second abutting position of the nipple (113a) in said opening (123a) corresponds to the putting of the fixing and reading chamber (103) into communication with the discharge chamber (104) and to the isolating of the inlet chamber (102).

6. Device according to any one of the preceding claims, **characterized in that** the cover (120) comprises a nipple (123b) on the external face of one of its coaxial cylindrical walls (123) which is situated outside the other cylindrical walls so as to form the external peripheral edge of the receptacle, said nipple (123b) forming a grip or a sill for turning the cover (120) about the vertical axis (101) with respect to the container (110).

7. Device according to any one of the preceding claims, **characterized in that** the receptacle comprises a central through-orifice (105) isolated from the immediately adjacent annular chamber (102) and intended to be threaded onto a vertical shaft for rotational driving for setting said receptacle into rotation.

8. Device according to any one of the preceding claims, **characterized in that** in the bottom (120a) of the cover (120) or in the bottom (110a) of the container (110), it comprises an entry orifice (102a) to the inlet chamber (102).

9. Device according to any one of Claims 1 to 8, **characterized in that** between the inlet chamber and the discharge chamber the receptacle comprises several concentric fixing and reading chambers.

10. Device according to any one of the preceding claims, **characterized in that** said coaxial cylindrical walls (111, 112; 121, 122) forming separations between the successive annular chambers (102, 103, 104) each comprise three openings (111a, 111b, 111c, 112a, 112b, 112c; 121a, 121b, 121c, 122a, 122b, 122c) regularly distributed over their circumference.

11. Device according to any one of the preceding claims, **characterized in that** the receptacle exhibits the general shape of a disc.

12. Device according to any one of the preceding claims, **characterized in that** the container (110) is made from a transparent material so as to allow reading of the labelled fixed components in the fixing and reading chamber through the walls of said receptacle.

13. Device according to any one of Claims 1 to 12, **characterized in that** it is made from plastic.

14. Device according to one of Claims 1 to 13, **characterized in that** in the fixing chamber (103) it comprises at least one receptor of the component to be dosed, said receptor being fixed on at least one of the walls of the chamber.

15. Device according to one of Claims 1 to 14, **characterized in that** said receptor is fixed on the bottom wall (110a) of the container (110).

16. Device according to one of Claims 1 to 15, **characterized in that** the inlet chamber (102) comprises a labelling element capable of labelling the component to be dosed.

17. Device according to one of Claims 1 to 16, **characterized in that** the fixed receptor is chosen from among:
- antibodies, antigens, complementary nucleic acid sequences, true receptors for respectively dosing components which are:
- antigens, antibodies, and nucleic acid sequences, ligands of the receptors.

18. Device according to any one of Claims 1 to 17, **characterized in that** the fixing and reading chamber (103) is divided into a plurality of angular sectors on which are fixed mutually different receptors each intended for fixing and for reading a different labelled component.

19. Device according to Claim 18, **characterized in that** an angular sector of the fixing chamber is left devoid of receptors so as to constitute a blank sector intended for carrying out an initialization reading of said device.

20. Device according to either of Claims 16 and 17, **characterized in that** the labelling is performed by physical or chemical means.

21. Device according to Claim 20, **characterized in that** the labelling is performed with the aid of fluorescent particles and/or elements or ones which can be rendered fluorescent.

22. Device according to Claim 21, **characterized in that** the particles exhibit a sufficient diameter, greater than or equal to around 100 times the diameter of the component(s) to be dosed and possess optical properties allowing their detection by counting.

23. Device according to Claim 21, **characterized in that** the particles have a diameter of 2 µm.

24. Device according to one of Claims 16 to 23, **characterized in that** the labelling element consists of a labelled antibody or a labelled antigen, or a labelled nucleic acid sequence or any receptor element which can be labelled.

25. Device according to one of the preceding claims, **characterized in that** the cover is treated in such a way as to avoid parasitic radiations and **in that** the reading is performed with the aid of a CCD camera.

26. Device according to Claim 25, **characterized in that** the CCD camera is able to count in a discretionary manner by emission/reception of a light signal, the number of labelled fixed components in each fixing and reading chamber, so as to obtain a digital detection signal.

27. Process for the qualitative and/or quantitative dosing of at least one particular component in a product sample by labelling and fixing, **characterized in that** use is made of at least one device according to any one of Claims 1 to 26 which contains specific receptors for the component to be dosed, which are fixed in each fixing and reading chamber and in which process,
a) the product sample containing the labelled component is placed in the inlet chamber isolated from the other annular chambers,
b) the cover is turned with respect to the container in such a way as to put the inlet chamber into communication with the fixing and reading chamber, the discharge chamber being isolated from the other annular chambers,
c) the device is rotated about its vertical axis in such a way as to disperse by centrifugation in each fixing and reading chamber the product sample containing the labelled component, the latter then binding by strong interaction to the fixed specific receptors in each fixing and reading chamber,
d) the cover is turned with respect to the container in such a way as to put the fixing and reading chamber into communication with the discharge chamber,
e) the device is rotated about its axis in such a way as to disperse by centrifugation the surplus sample into the discharge chamber,
f) the inside of the device is rinsed with the aid of a rinsing liquid which is circulated by centrifugation through the various annular chambers of said device while reproducing the preceding steps b), c), d) and e) in such a way as to retain in the fixing and reading chamber only the labelled component bound by strong interaction to the fixed receptors,
g) said labelled component is detected and dosed through the wall(s) of said device by an appropriate means.

28. Process according to Claim 27, **characterized in that** the labelling of each particular component to be dosed of the product sample is carried out outside before introducing the latter into the inlet chamber of the device.

29. Process according to Claim 27, **characterized in that** the labelling of each component to be dosed of the product sample is carried out in the inlet and labelling chamber, by previously introducing into the product sample a specific labelling element for each component to be dosed, preferably in unfixed dry form, then by introducing the product sample into said isolated inlet chamber so that the labelling element will bind by strong interaction to the corresponding component contained in said product sample.

30. Process according to one. of Claims 27 to 29, **characterized in that** the reading operation allowing the detection of the labelled fixed component in the fixing and reading chamber is carried out with the aid of a CCD camera.

31. Process according to Claim 30, **characterized in that** the CCD camera carries out a discretionary counting by emission/reception of a light signal of the labelled fixed components in each fixing and reading chamber, so as to obtain a digital detection signal.

32. Process according to one of Claims 27 to 31, **characterized in that** the reading of the labelled fixed agents is performed according to radii of the fixing and reading chamber.

33. Apparatus for implementing the process according to one of Claims 27 to 32, **characterized in that** it comprises a vertical shaft for rotational driving on which are threaded devices according to one of Claims 1 to 26, means for maintaining said devices distanced from one another, means for the bidirectional rotational driving of said vertical drive shaft, means for injecting product samples and the rinsing liquid into the inlet chambers of said devices threaded on the drive shaft, and means for turning the covers of the devices relative to the containers in such a way as to put the various successive annular chambers of these containers into communication or to isolate them and a means of reading the labelled fixed agents.
